# EUROPEAN PATENT APPLICATION

(11) **EP 4 649 975 A1**
(43) Date of publication of application: **19.11.2025**
(21) Application number: 23917328.9
(22) Date of filing: 27.12.2023
(51) Int. Cl.: A61M 5/178, A61M 5/315

(54) **QUANTITATIVE INJECTION SYRINGE**

(30) Priority: 16.01.2023 CN 202310062577; 25.06.2023 CN 202310751858
(71) Applicant: Berpu Medical Technology Co., Ltd., Wenzhou, Zhejiang 325024 (CN)
(72) Inventor: ZHANG, Hongjie, Wenzhou, Zhejiang 325024 (CN); ZHANG, Ce, Wenzhou, Zhejiang 325024 (CN)
(74) Representative: Bayramoglu et al.
(86) International application number: PCT/CN2023/142525
(87) International publication number: WO 2024/152863

(57) **Abstract**

A quantitative injection syringe includes two pressure blocks (1), an outer sleeve (2), and a core rod (3). By providing the two pressure blocks (1) on the outer sleeve (2) for limiting snap-fitting to the core rod (3). The volume of liquid medicine input into the outer sleeve (2) can be defined during liquid medicine suctioning and a first-level volume of liquid medicine injected can be defined during liquid medicine injection, so that the objective of controlling an injection amount is achieved, and the problem of a medical accident caused by a too large one-time injection amount is avoided, and the position of the core rod (3) relative to the pressure blocks (1) can be subsequently adjusted to further cause the core rod (3) to be pushed into the outer sleeve (2) to inject the remaining liquid medicine to define a second-level volume of liquid medicine injected, thereby effectively grasping the total injection amount of liquid medicine.

## Description

### TECHNICAL FIELD

The present application relates to the field of medical devices, and specifically to a quantitative syringe.

### BACKGROUND ART

Syringes are instruments used for injecting or extracting liquids. Typically, syringes have graduated markings to indicate the volume of liquid inside.

Existing syringes rely on manual observation of the graduations to determine the injection volume, which is highly susceptible to human error. Over-injection may occur, leading to excessive single-dose administration and potential medical accidents.

Therefore, current syringe structures lack effective mechanisms to limit injection volume, making it difficult to control dosage accurately. This may result in excessive single-dose injections, posing risks of medical incidents.

### CONTENT OF THE INVENTION

The embodiments of the present application provide a quantitative syringe with a limiting structure to control injection volume, addressing the technical problem of existing syringe structures lacking precise volume limitation, which may lead to over-injection and medical accidents.

The embodiments of the present application provide a quantitative syringe, including two pressing blocks, an outer sleeve and a core rod;
wherein the core rod is slidably arranged inside the outer sleeve, the core rod is provided with a push handle, a rod body and a push head; the push handle is arranged outside the outer sleeve, the push head is located inside the outer sleeve, the rod body is located inside the outer sleeve and is connected at both ends to the push handle and the push head respectively, the rod body is provided with a first limiting structure at an end adjacent to the push head, and a second limiting structure at an end adjacent to the push handle, with a gap provided between the first limiting structure and the second limiting structure;
the two pressing blocks are arranged at an end of the outer sleeve adjacent to the push handle and are snap-fitted onto the outer sleeve, the two pressing blocks are provided with an inner blocking surface for engaging with the first limiting structure and an outer blocking surface for engaging with the second limiting structure; and
when the core rod is pulled out of the outer sleeve to draw in liquid medicine, the inner blocking surface blocks the first limiting structure to limit the volume of liquid medicine drawn into the outer sleeve; when the core rod is pushed into the outer sleeve to inject the liquid medicine, the outer blocking surface blocks the second limiting structure to limit a first-stage injection volume of the liquid medicine.

Further, the first limiting structure is a limiting step, the second limiting structure is a limiting rib; the two pressing blocks are oppositely arranged to form a cross-shaped guide groove, the rod body is a cross-shaped guide rod, and the cross-shaped guide rod matches the cross-shaped guide groove.

Further, the cross-shaped guide groove includes a first guide groove and a second guide groove, a depth direction of the first guide groove is perpendicular to a depth direction of the second guide groove, a depth of the first guide groove matches a thickness of the cross-shaped guide rod, and a depth of the second guide groove matches a sum of the thicknesses of the cross-shaped guide rod and the limiting rib.

Further, when the core rod is pushed into the outer sleeve to inject the liquid medicine, the limiting rib corresponds to the first guide groove, and an edge of the first guide groove serves as the outer blocking surface.

Further, four protrusions are provided between the first guide groove and the second guide groove, the core rod is rotatable to squeeze and deform the protrusions so as to switch the limiting rib from corresponding to the first guide groove to corresponding to the second guide groove; when the limiting rib corresponds to the second guide groove, the limiting rib is slidable along the second guide groove, and the core rod is pushed into the outer sleeve to inject the remaining liquid medicine to limit a second-stage injection volume of the liquid medicine.

Further, the protrusions are provided with squeeze holes; when the core rod rotates to squeeze and deform the protrusions, the squeeze holes deform accordingly.

Further, the two pressing blocks are symmetrically hinged to an end of the outer sleeve adjacent to the push handle via connecting ribs; the outer sleeve is provided with two clamping platforms, each clamping platform is provided with a positioning hole; each pressing block is provided with a positioning post facing the positioning hole of the clamping platform, and the positioning post is engaged in the positioning hole.

Further, two symmetrically arranged snap-through holes are provided on both side edges of each clamping platform, each pressing block is provided with a hook facing the snap-through hole of the clamping platform, and the hook passes through the snap-through hole and is engaged therein.

Further, the quantitative syringe further includes a piston, the piston is arranged on an outer side of the push head, and the piston is in interference fit with an inner wall of the outer sleeve.

Further, the quantitative syringe further includes a needle tube and a needle protector; the needle tube is connected to an end of the outer sleeve and corresponds to the push head; the needle protector is sleeved on the end of the outer sleeve, and the needle tube is arranged inside the needle protector.

Further, the rod body is further provided with auxiliary limiting ribs, the auxiliary limiting ribs are arranged between the limiting step and the limiting rib; two auxiliary limiting ribs are provided, two limiting ribs are provided, the cross-shaped guide rod is provided with four convex ribs of the same thickness, the two auxiliary limiting ribs are symmetrically arranged on two convex ribs of the cross-shaped guide rod, and the two limiting ribs are symmetrically arranged on the other two convex ribs of the cross-shaped guide rod; the auxiliary limiting ribs and the limiting ribs are alternately arranged along a circumferential direction of the cross-shaped guide rod, and a rotational gap is provided between a distribution region of the auxiliary limiting ribs along a length direction of the cross-shaped guide rod and a distribution region of the limiting ribs along the length direction of the cross-shaped guide rod.

Further, multiple distribution regions of the auxiliary limiting ribs are provided along the length direction of the cross-shaped guide rod, each distribution region of the auxiliary limiting ribs is provided with two symmetrically arranged auxiliary limiting ribs, a rotational gap is provided between adjacent distribution regions of the auxiliary limiting ribs, and the auxiliary limiting ribs in adjacent distribution regions are arranged on two sets of convex ribs of the cross-shaped guide rod.

Further, the two pressing blocks are symmetrically hinged to an end of the outer sleeve adjacent to the push handle, or the two pressing blocks are separately arranged from the outer sleeve.

The embodiments of the present application provide a precise and easy-to-operate quantitative syringe. By arranging two pressing blocks on the outer sleeve to limit and engage with the core rod, it achieves the objectives of limiting the intake volume when drawing liquid medicine and limiting the first-stage injection volume when injecting liquid medicine, thereby realizing precise dosage control and avoiding medical accidents caused by excessive single-dose injections. Moreover, by adjusting the relative position between the core rod and the pressing blocks, the core rod can be further pushed into the outer sleeve to inject the remaining liquid medicine, thereby limiting the second-stage injection volume and effectively controlling the total injection volume.

### DESCRIPTION OF THE DRAWINGS

To more clearly illustrate the technical solutions in the embodiments of the present application, the following briefly introduces the accompanying drawings required for describing the embodiments. It should be understood that the following drawings show only some embodiments of the present application and therefore should not be considered as limiting the scope.
FIG. 1 is a structural schematic diagram of the quantitative syringe provided by an embodiment of the present application.
FIG. 2 is a structural schematic diagram of the quantitative syringe in FIG. 1 after drawing liquid medicine.
FIG. 3 is an exploded view of part of the structure of the quantitative syringe in FIG. 2.
FIG. 4 is a structural schematic diagram of the quantitative syringe in an embodiment of the present application in a state of injecting the first-stage liquid medicine volume.
FIG. 5 is a schematic diagram of the rotation principle of the core rod corresponding to FIG. 4.
FIG. 6 is a structural schematic diagram of the quantitative syringe in an embodiment of the present application in a state of injecting the second-stage liquid medicine volume.
FIG. 7 is a schematic diagram of the rotation principle of the core rod corresponding to FIG. 6.
FIG. 8 is a structural schematic diagram of the core rod, pressing blocks and outer sleeve of the quantitative syringe in an embodiment of the present application when not snap-fitted.
FIG. 9 is a structural schematic diagram of the core rod, pressing blocks and outer sleeve of the quantitative syringe in an embodiment of the present application when snap-fitted.
FIG. 10 is a structural schematic diagram of the core rod, pressing blocks and outer sleeve of the quantitative syringe in an embodiment of the present application after snap-fitting.
FIG. 11 is a structural schematic diagram of the positioning post of the pressing block engaged with the positioning hole of the outer sleeve after snap-fitting of the quantitative syringe in an embodiment of the present application.
FIG. 12 is a structural schematic diagram of the hook of the pressing block engaged with the snap-through hole after snap-fitting of the quantitative syringe in an embodiment of the present application.
FIG. 13 is a structural schematic diagram of another quantitative syringe provided by an embodiment of the present application.
FIG. 14 is a structural schematic diagram of segment switching of the quantitative syringe provided by an embodiment of the present application.
FIG. 15 is a structural schematic diagram of yet another quantitative syringe provided by an embodiment of the present application.

The labels in the figures are as follows:
Pressing block 1, cross-shaped guide groove 10, first guide groove 101, second guide groove 102, protrusion 103, squeeze hole 104, connecting rib 11, positioning post 12, hook 13, inner blocking surface 110, outer blocking surface 120,
Outer sleeve 2, clamping platform 21, positioning hole 211, snap-through hole 212,
Core rod 3, push handle 31, rod body 32, push head 33, limiting step 34, limiting rib 35, auxiliary limiting rib 36, rotational gap 37,
Piston 4, needle tube 5, needle protector 6.

### SPECIFIC IMPLEMENTATIONS

The technical solutions in the embodiments of the present application will be clearly and completely described below with reference to the accompanying drawings in the embodiments of the present application. Obviously, the described embodiments are only some of the embodiments of the present application, rather than all of them. Based on the embodiments in the present application, all other embodiments obtained by those of ordinary skill in the art without creative efforts shall fall within the protection scope of the present application.

In the description of the present application, it should be noted that the terms "installation", "connected", and "connection" should be understood broadly, unless otherwise expressly specified and limited. For example, it can be a fixed connection, a detachable connection, or an integral connection; it can be a mechanical connection, an electrical connection, or a communication connection; it can be a direct connection, or an indirect connection through an intermediate medium, or an internal communication between two elements or an interaction relationship between two elements. For those of ordinary skill in the art, the specific meanings of the above terms in the present application can be understood according to specific circumstances.

### Embodiment 1

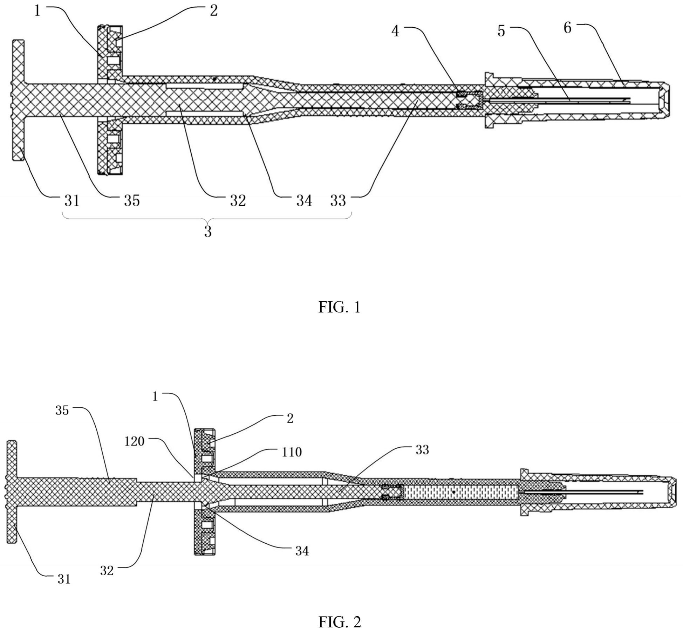

Please refer to FIGS. 1 to 8. Embodiment 1 of the present application provides a quantitative syringe. The quantitative syringe is precise and easy to operate, with features such as extremely easy operation, foolproof design, and precise quantitative injection.

Please refer to FIGS. 1, 2, and 3. The quantitative syringe provided by the embodiments of the present application includes two pressing blocks 1, an outer sleeve 2, and a core rod 3.

Among them, the core rod 3 is slidably arranged inside the outer sleeve 2. The core rod 3 is provided with a push handle 31, a rod body 32, and a push head 33. The push handle 31 is arranged outside the outer sleeve 2, the push head 33 is located inside the outer sleeve 2, and the rod body 32 is located inside the outer sleeve 2 and is connected at both ends to the push handle 31 and the push head 33 respectively. A first limiting structure is provided at an end of the rod body 32 adjacent to the push head 33, and the first limiting structure is preferably a limiting step 34. A second limiting structure is provided at an end of the rod body 32 adjacent to the push handle 31, and the second limiting structure is preferably a limiting rib 35. A gap is provided between the first limiting structure and the second limiting structure, that is, a gap is provided between the limiting step 34 and the limiting rib 35.The two pressing blocks 1 are symmetrically hinged to an end of the outer sleeve 2 adjacent to the push handle 31 and are snap-fitted onto the outer sleeve 2. The two pressing blocks 1 are provided with an inner blocking surface 110 for engaging with the limiting step 34 (first limiting structure) and an outer blocking surface 120 for engaging with the limiting rib 35 (second limiting structure). Please refer to FIG. 2. When the core rod 3 is pulled out of the outer sleeve 2 to draw liquid medicine, the inner blocking surface 110 blocks the limiting step 34 (first limiting structure) to limit the volume of liquid medicine drawn into the outer sleeve 2. When the limiting step 34 (first limiting structure) contacts the inner blocking surface 110, it indicates that the liquid drawing has reached the set capacity.

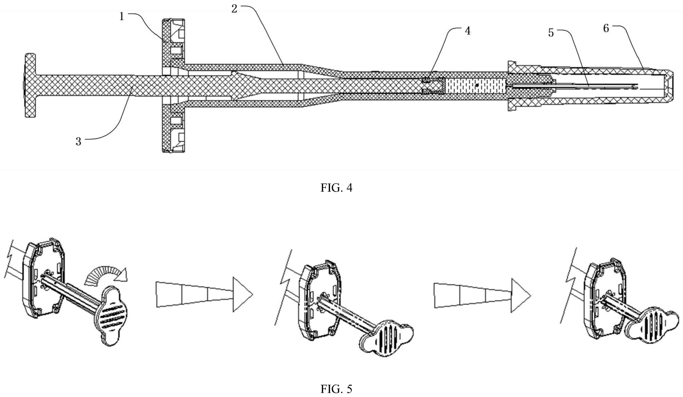

Please refer to FIG. 4. When the core rod 3 is pushed into the outer sleeve 2 to inject liquid medicine, the outer blocking surface 120 blocks the limiting rib 35 (second limiting structure) to limit the first-stage injection volume of the liquid medicine.

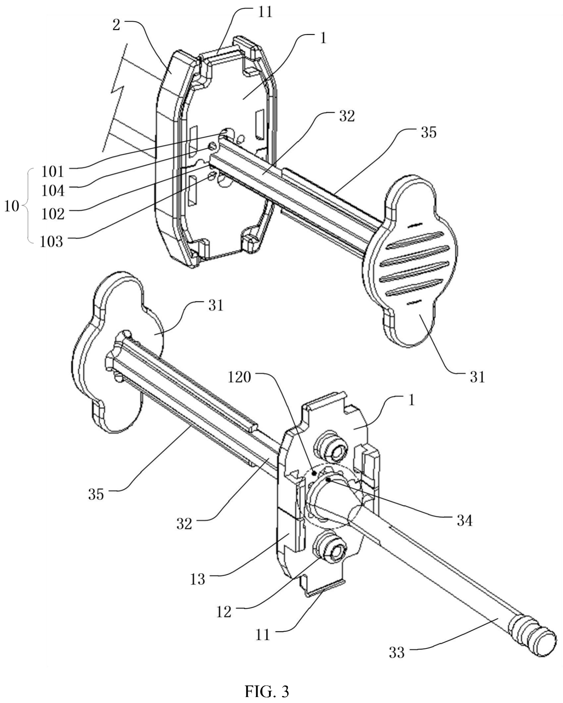

Please refer to FIG. 3. The two pressing blocks 1 are oppositely arranged to form a cross-shaped guide groove 10. The rod body 32 is a cross-shaped guide rod, and the cross-shaped guide rod matches the cross-shaped guide groove 10, allowing the core rod 3 to slide smoothly inside the outer sleeve 2 without jamming or tilting.

Please refer to FIG. 3. The cross-shaped guide groove 10 includes a first guide groove 101 and a second guide groove 102. The depth direction of the first guide groove 101 is perpendicular to the depth direction of the second guide groove 102. The depth of the first guide groove 101 matches the thickness of the cross-shaped guide rod, and the depth of the second guide groove 102 matches the sum of the thicknesses of the cross-shaped guide rod and the limiting rib 35.

Please refer to FIG. 3. When the core rod 3 is pushed into the outer sleeve 2 to inject liquid medicine, the limiting rib 35 corresponds to the first guide groove 101, and the edge of the first guide groove 101 serves as the outer blocking surface 120.

Please refer to FIG. 3. Four protrusions 103 are provided between the first guide groove 101 and the second guide groove 102. The core rod 3 can be rotated to squeeze and deform the protrusions 103, thereby switching the limiting rib 35 from corresponding to the first guide groove 101 to corresponding to the second guide groove 102. When the limiting rib 35 corresponds to the second guide groove 102, the limiting rib 35 can slide along the second guide groove 102, and the core rod 3 is pushed into the outer sleeve 2 to inject the remaining liquid medicine, thereby limiting the second-stage injection volume of the liquid medicine.

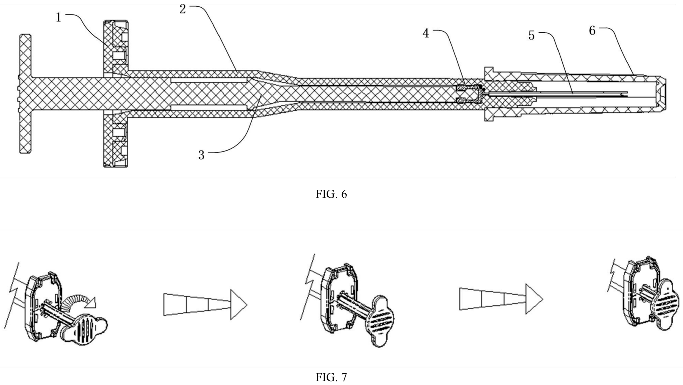

Please refer to FIG. 3. The protrusions 103 are provided with squeeze holes 104. Please refer to FIGS. 5 and 7. When the core rod 3 rotates to squeeze and deform the protrusions 103, the squeeze holes 104 deform accordingly.

It can be understood that the cross-shaped guide rod has four concave guide grooves and four convex guide rails. The two pressing blocks 1 are provided with four protrusions 103 in the cross-shaped guide groove 10, and each protrusion 103 is provided with a squeeze hole 104. The protrusions 103 cooperate with the concave guide grooves, and the first guide groove 101 and the second guide groove 102 cooperate with the convex guide rails. When starting the first-stage liquid injection, first rotate the core rod 3. During rotation, the four convex guide rails of the core rod 3 squeeze the four protrusions 103 of the pressing blocks 1, causing the protrusions 103 to deform, and the squeeze holes 104 also deform accordingly. After rotating past the protrusions 103, the four convex guide rails of the core rod 3 fall into the concave grooves of the pressing blocks 1, and the four protrusions 103 of the pressing blocks 1 again provide limiting guidance. At this time, push the core rod 3 to compress and output the liquid. The limiting rib 35 of the core rod 3 will contact the outer blocking surface 120 of the pressing block 1, completing the first-stage liquid injection according to the set capacity. Rotating the core rod 3 provides a tactile feedback of squeezing and positioning, and the limiting rib 35 determines the first-stage output capacity.

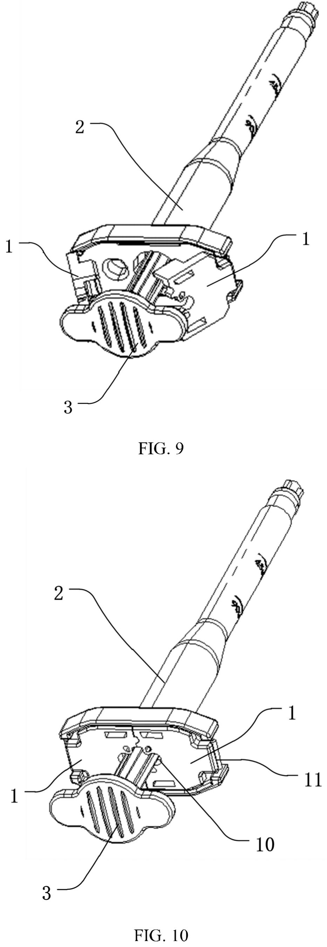

Please refer to FIGS. 3, 8, 9, 10, 11, and 12. The two pressing blocks 1 are symmetrically hinged to an end of the outer sleeve 2 adjacent to the push handle 31 via connecting ribs 11. The connecting ribs 11 are flexible and can be flipped, connecting the two pressing blocks 1 and the outer sleeve 2 as a whole. The outer sleeve 2 is provided with two clamping platforms 21, and each clamping platform 21 is provided with a positioning hole 211. Each pressing block 1 is provided with a positioning post 12 facing the positioning hole 211 of the clamping platform 21, and the positioning post 12 is engaged in the positioning hole 211.

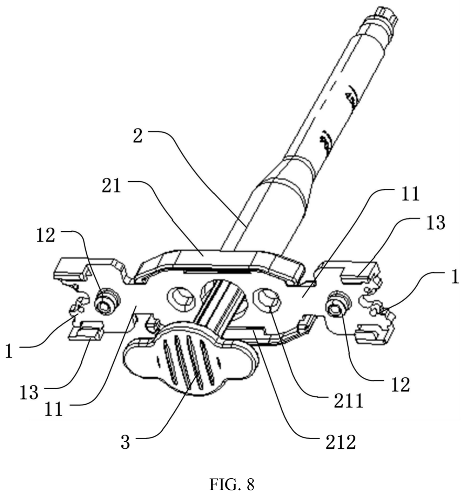

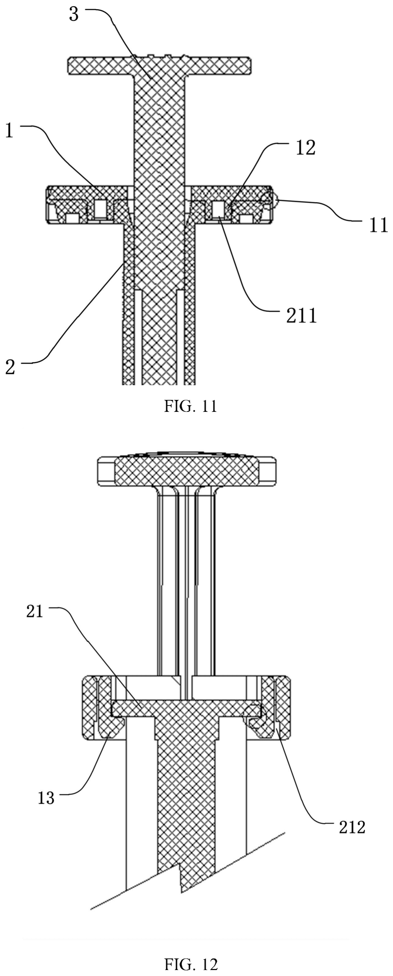

Please refer to FIGS. 8 and 12. Two symmetrically arranged snap-through holes 212 are provided on both side edges of each clamping platform 21. Each pressing block 1 is provided with a hook 13 facing the snap-through hole 212 of the clamping platform 21, and the hook 13 passes through the snap-through hole 212 and is engaged therein.

Specifically, the outer sleeve 2 and the two pressing blocks 1 are injection-molded as one part. During assembly, flip the pressing blocks 1, causing the connecting ribs 11 to deform. The hooks 13 of the pressing blocks 1 pass through the snap-through holes 212 of the outer sleeve 2 and hook onto the clamping platforms 21 to prevent detachment. At the same time, the positioning posts of the pressing blocks 1 enter the positioning holes of the outer sleeve 2. In this way, the pressing blocks 1 are accurately positioned and not easily detached.

Please refer to FIG. 1. The quantitative syringe further includes a piston 4. The piston 4 is arranged on the outer side of the push head 33 and is in interference fit with the inner wall of the outer sleeve 2.

It can be understood that the piston 4 is provided with an end surface, and the outer sleeve 2 is provided with a core bottom surface. The second guide groove 102 is a large concave groove relative to the first guide groove 101. When starting the second-stage liquid injection, first rotate the core rod 3. During rotation, the four convex guide rails of the core rod 3 squeeze the four protrusions 103 of the pressing blocks 1, causing the protrusions 103 to deform, and the squeeze holes 104 also deform accordingly. After rotating past the protrusions 103, the four convex guide rails of the core rod 3 fall into the second guide grooves 102 of the pressing blocks 1, and the four protrusions 103 of the pressing blocks 1 again provide limiting guidance. At this time, the limiting rib 35 avoids the outer blocking surface 120 and falls into the large concave groove of the pressing block 1. Push the core rod 3 to compress and output the liquid for the second time. The end surface of the piston 4 contacts the core bottom surface of the outer sleeve 2, completing the second-stage liquid injection.

Please refer to FIG. 1. The quantitative syringe further includes a needle tube 5 and a needle protector 6. The needle tube 5 is connected to an end of the outer sleeve 2 and corresponds to the push head 33. The needle protector 6 is sleeved on the end of the outer sleeve 2, and the needle tube 5 is arranged inside the needle protector 6.

Please refer to FIGS. 2, 4, 5, 6, and 7. When using the quantitative syringe, the pressing blocks 1 are fixed on the outer sleeve 2 at the flanged end. The pressing blocks 1 are provided with a cross-shaped guide groove 10 at the center to form a limiting through hole. When pulling the core rod 3 outward to draw liquid medicine, the cross-shaped guide rod of the core rod 3 can easily pass over the pressing blocks 1 on the outer sleeve 2 via guide slopes. At the same time, by rotating and adjusting the position of the core rod 3, the profile of the cross-shaped guide rod of the core rod 3 can precisely pass through the limiting through hole at the center of the pressing blocks 1. At this time, the rotation angle of the core rod 3 requires high precision. When pushing the core rod 3 inward to inject liquid medicine, rotate and adjust the position of the core rod 3. The cross-shaped guide rod of the core rod 3 cannot easily pass over the elastic hooks 13 of the outer sleeve 2, and the rotation angle adjustment of the core rod 3 is relatively easy at this time. This reminds the operator that the syringe has injected a certain amount of liquid medicine, and this dose is a specially set dose. An identifier such as an arrow, dot, or graduation line is provided at this stopping position. To continue the injection, rotate and adjust the position of the core rod 3 again for the next injection step. In this way, over-injection can be prevented, the injection volume of the syringe can be controlled, precise extraction or transfer of drugs can be achieved, the detection accuracy of related instruments can be improved, and medical accidents can be prevented.

### Embodiment 2

Embodiment 2 of the present application includes most of the technical features of Embodiment 1, with the difference being that Embodiment 2 does not include the squeeze holes 104 of Embodiment 1, that is, the protrusions 103 are not provided with squeeze holes 104. The protrusions 103 are preferably made of flexible and elastic material. When the core rod 3 rotates to squeeze and deform the protrusions 103, even without squeeze holes 104, the rotation of the rod body 32 in the cross-shaped guide groove 10 can still be achieved.

The specific content of this embodiment refers to the content of Embodiment 1 and will not be repeated here.

### Embodiment 3

Embodiment 3 of the present application includes most of the technical features of Embodiment 1, with the difference being that this embodiment can achieve multi-stage limiting.

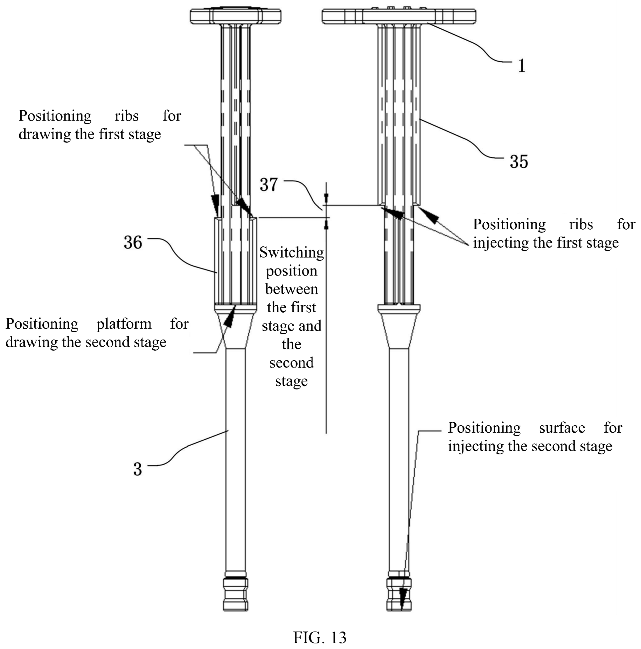

As shown in FIG. 13, the rod body 32 is further provided with auxiliary limiting ribs 36. The auxiliary limiting ribs 36 are arranged between the limiting step 34 and the limiting rib 35. Two auxiliary limiting ribs 36 are provided, and two limiting ribs 35 are provided. The cross-shaped guide rod is provided with four convex ribs of the same thickness. The two auxiliary limiting ribs 36 are symmetrically arranged on two convex ribs of the cross-shaped guide rod, and the two limiting ribs 35 are symmetrically arranged on the other two convex ribs of the cross-shaped guide rod. The auxiliary limiting ribs 36 and the limiting ribs 35 are alternately arranged along the circumferential direction of the cross-shaped guide rod, and a rotational gap 37 is provided between the distribution region of the auxiliary limiting ribs 36 along the length direction of the cross-shaped guide rod and the distribution region of the limiting ribs 35 along the length direction of the cross-shaped guide rod.

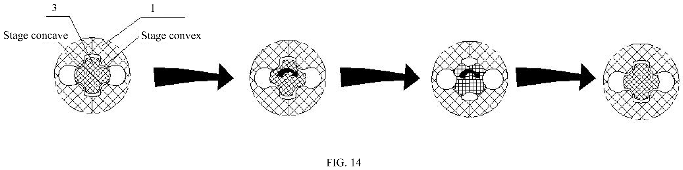

As shown in FIG. 14, the depths of the first guide groove 101 and the second guide groove 102 are different. For example, the depth of the first guide groove 101 is greater than that of the second guide groove 102. When the limiting rib 35 corresponds to the first guide groove 101, the core rod 3 can slide along the length direction of the outer sleeve 2 to achieve liquid medicine drawing or injection. When the auxiliary limiting rib 36 abuts against the outer sleeve 2, the auxiliary limiting rib 36 serves as a limiting function. At this time, the core rod 3 can be rotated to align the auxiliary limiting rib 36 with the first guide groove 101. At this time, the core rod 3 can further slide along the length direction of the outer sleeve 2, and the limiting rib 35 abuts against the outer sleeve 2 to serve as a limiting function. The two symmetrically arranged end surfaces are located in the same plane to form a positioning circular platform.

It can be understood that both end surfaces along the length direction of the limiting rib 35 and the auxiliary limiting rib 36 protrude from the outer side surface of the core rod 3. The end surfaces along the length direction of the limiting rib 35 and the auxiliary limiting rib 36 achieve limiting functions, completing the limiting use effect of a single-stage dose. The rotational gap 37 provides a rotation range for the rod body 32 of the core rod 3 in the cross-shaped guide groove 10.

Further, multiple distribution regions of the auxiliary limiting ribs 36 are provided along the length direction of the cross-shaped guide rod. Each distribution region of the auxiliary limiting ribs 36 is provided with two symmetrically arranged auxiliary limiting ribs 36. A rotational gap 37 is provided between adjacent distribution regions of the auxiliary limiting ribs 36. The auxiliary limiting ribs 36 in adjacent distribution regions are arranged on two sets of convex ribs of the cross-shaped guide rod.

Here, two convex ribs arranged in a straight line and symmetrically arranged form one set. The other pair of convex ribs arranged in a cross shape on the cross-shaped guide rod form another set.

This embodiment provides users with the option of using one stage or two stages or even multiple stages. For example, when only one stage is used, only one stage is drawn for direct injection. When two stages are needed, only one stage is drawn first, then the core rod 3 is rotated to draw the second stage, the first stage is injected, and then the core rod 3 is rotated to inject the second stage. Alternatively, by setting multiple structures for switching between the first stage and the second stage, users can be provided with the option of multiple stages.

In addition, the outer sleeve 2 is provided with integrated pressing blocks 1, which can be flipped and hooked for assembly. The upper and lower surfaces serve as positioning for the first stage, second stage, or even multiple stages. The pressing blocks 1 are provided with cross-shaped through holes and protrusions, allowing the core rod 3 to slide directionally and providing tactile feedback during stage switching. The core rod 3 is provided with positioning ribs for drawing the first stage and injecting the first stage, and also has a positioning circular platform for drawing the second stage. The core rod 3 is in a cross shape, allowing directional sliding in the outer sleeve and providing obvious tactile feedback when switching between the first stage and the second stage, facilitating operation.

### Embodiment 4

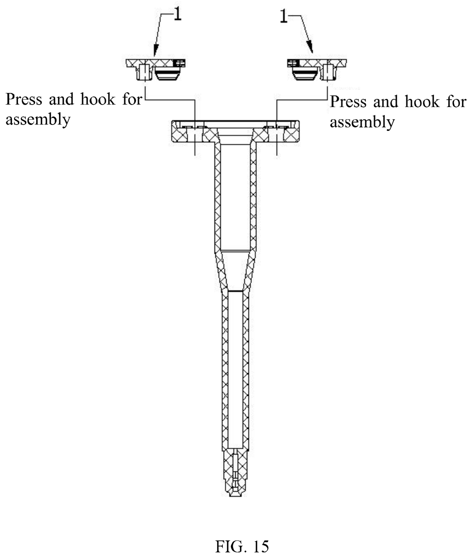

As shown in FIG. 15, Embodiment 4 of the present application includes most of the technical features of Embodiments 1-3, with the difference being that the two pressing blocks 1 are separately arranged from the outer sleeve 2.

The outer sleeve 2 and the separate positioning pressing blocks 1 can be directly hooked and assembled. The upper and lower surfaces serve as positioning for the first stage, second stage, or even multiple stages. The pressing blocks 1 are provided with cross-shaped through holes and protrusions, allowing the core rod 3 to slide directionally and providing tactile feedback during stage switching, facilitating operation.

The embodiments of the present application provide a precise and easy-to-operate quantitative syringe. By arranging two pressing blocks on the outer sleeve to limit and engage with the core rod, it achieves the objectives of limiting the intake volume when drawing liquid medicine and limiting the first-stage injection volume when injecting liquid medicine, thereby realizing precise dosage control and avoiding medical accidents caused by excessive single-dose injections. Moreover, by adjusting the relative position between the core rod and the pressing blocks, the core rod can be further pushed into the outer sleeve to inject the remaining liquid medicine, thereby limiting the second-stage injection volume and effectively controlling the total injection volume.

The embodiments of the present application also have the following additional advantages:
1. High precision for small-bore injection administration;
2. Stable and controllable limiting structure to ensure administration accuracy;
3. Relatively simple structure with low cost;
4. Low residual design;
5. The core rod moves along a set track, providing foolproof guidance and tactile feedback;
6. The core rod provides tactile feedback when adjusting the injection dose, with strong recognizability.

In the above embodiments, the descriptions of the various embodiments have their own emphases. For parts not described in detail in a certain embodiment, reference may be made to the relevant descriptions of other embodiments.

The above provides a detailed description of a quantitative syringe provided by the embodiments of the present application. Specific examples are used in this specification to explain the principles and implementation of the present application. The descriptions of the above embodiments are only used to help understand the technical solutions and core ideas of the present application. Those of ordinary skill in the art should understand that they can still modify the technical solutions described in the foregoing embodiments or equivalently replace some of the technical features. These modifications or replacements do not cause the essence of the corresponding technical solutions to deviate from the scope of the technical solutions of the embodiments of the present application.

## Claims

1. A quantitative syringe, **characterized by** comprising two pressing blocks, an outer sleeve, and a core rod;
wherein the core rod is slidably arranged inside the outer sleeve, the core rod is provided with a push handle, a rod body, and a push head; the push handle is arranged outside the outer sleeve, the push head is located inside the outer sleeve, the rod body is located inside the outer sleeve and is connected at both ends to the push handle and the push head, respectively; a first limiting structure is provided at an end of the rod body adjacent to the push head, a second limiting structure is provided at an end of the rod body adjacent to the push handle, and a gap is provided between the first limiting structure and the second limiting structure;
the two pressing blocks are arranged at an end of the outer sleeve adjacent to the push handle and are snap-fitted onto the outer sleeve, the two pressing blocks are provided with an inner blocking surface for engaging with the first limiting structure and an outer blocking surface for engaging with the second limiting structure; and
when the core rod is pulled out of the outer sleeve to draw in liquid medicine, the inner blocking surface blocks the first limiting structure to limit the volume of liquid medicine drawn into the outer sleeve; when the core rod is pushed into the outer sleeve to inject the liquid medicine, the outer blocking surface blocks the second limiting structure to limit a first-stage injection volume of the liquid medicine.

2. The quantitative syringe according to claim 1, **characterized in that** the first limiting structure is a limiting step, the second limiting structure is a limiting rib; the two pressing blocks are oppositely arranged to form a cross-shaped guide groove, the rod body is a cross-shaped guide rod, and the cross-shaped guide rod matches the cross-shaped guide groove.

3. The quantitative syringe according to claim 2, **characterized in that** the cross-shaped guide groove comprises a first guide groove and a second guide groove, a depth direction of the first guide groove is perpendicular to a depth direction of the second guide groove, a depth of the first guide groove matches a thickness of the cross-shaped guide rod, and a depth of the second guide groove matches a sum of the thicknesses of the cross-shaped guide rod and the limiting rib.

4. The quantitative syringe according to claim 3, **characterized in that** when the core rod is pushed into the outer sleeve to inject the liquid medicine, the limiting rib corresponds to the first guide groove, and an edge of the first guide groove serves as the outer blocking surface.

5. The quantitative syringe according to claim 4, **characterized in that** four protrusions are provided between the first guide groove and the second guide groove, the core rod is rotatable to squeeze and deform the protrusions so as to switch the limiting rib from corresponding to the first guide groove to corresponding to the second guide groove; when the limiting rib corresponds to the second guide groove, the limiting rib is slidable along the second guide groove, and the core rod is pushed into the outer sleeve to inject the remaining liquid medicine to limit a second-stage injection volume of the liquid medicine.

6. The quantitative syringe according to claim 5, **characterized in that** the protrusions are provided with squeeze holes; when the core rod rotates to squeeze and deform the protrusions, the squeeze holes deform accordingly.

7. The quantitative syringe according to claim 1, **characterized in that** the two pressing blocks are symmetrically hinged to an end of the outer sleeve adjacent to the push handle via connecting ribs; the outer sleeve is provided with two clamping platforms, each clamping platform is provided with a positioning hole; each pressing block is provided with a positioning post facing the positioning hole of the clamping platform, and the positioning post is engaged in the positioning hole.

8. The quantitative syringe according to claim 7, **characterized in that** two symmetrically arranged snap-through holes are provided on both side edges of each clamping platform, each pressing block is provided with a hook facing the snap-through hole of the clamping platform, and the hook passes through the snap-through hole and is engaged therein.

9. The quantitative syringe according to claim 1, **characterized in that** the quantitative syringe further comprises a piston, the piston is arranged on an outer side of the push head, and the piston is in interference fit with an inner wall of the outer sleeve.

10. The quantitative syringe according to claim 1, **characterized in that** the quantitative syringe further comprises a needle tube and a needle protector; the needle tube is connected to an end of the outer sleeve and corresponds to the push head; the needle protector is sleeved on the end of the outer sleeve, and the needle tube is arranged inside the needle protector.

11. The quantitative syringe according to claim 5, **characterized in that** the rod body is further provided with auxiliary limiting ribs, the auxiliary limiting ribs are arranged between the limiting step and the limiting rib; two auxiliary limiting ribs are provided, two limiting ribs are provided, the cross-shaped guide rod is provided with four convex ribs of the same thickness, the two auxiliary limiting ribs are symmetrically arranged on two convex ribs of the cross-shaped guide rod, and the two limiting ribs are symmetrically arranged on the other two convex ribs of the cross-shaped guide rod; the auxiliary limiting ribs and the limiting ribs are alternately arranged along a circumferential direction of the cross-shaped guide rod, and a rotational gap is provided between a distribution region of the auxiliary limiting ribs along a length direction of the cross-shaped guide rod and a distribution region of the limiting ribs along the length direction of the cross-shaped guide rod.

12. The quantitative syringe according to claim 11, **characterized in that** multiple distribution regions of the auxiliary limiting ribs are provided along the length direction of the cross-shaped guide rod, each distribution region of the auxiliary limiting ribs is provided with two symmetrically arranged auxiliary limiting ribs, a rotational gap is provided between adjacent distribution regions of the auxiliary limiting ribs, and the auxiliary limiting ribs in adjacent distribution regions are arranged on two sets of convex ribs of the cross-shaped guide rod.

13. The quantitative syringe according to claim 1, **characterized in that** the two pressing blocks are symmetrically hinged to an end of the outer sleeve adjacent to the push handle, or the two pressing blocks are separately arranged from the outer sleeve.
